# EUROPEAN PATENT SPECIFICATION

(11) **EP 0 925 770 B1**
(45) Date of publication and mention of the grant of the patent: **18.09.2002**
(21) Application number: 98310601.4
(22) Date of filing: 22.12.1998
(51) Int. Cl.: A61F 13/56, A61F 13/15, A61F 13/58

(54) **Fastening device of disposable diaper**
Verschluss für Wegwerfwindel
Dispositif de fixation de couche-culotte jetable

(30) Priority: 24.12.1997 JP 35440397
(43) Date of publication of application: 30.06.1999
(73) Proprietor: YKK CORPORATION, Chiyoda-ku, Tokyo (JP)
(72) Inventor: Yamamoto, Toru, Meerbusch 40667 (DE); Suenaga, Tomohiro, Ikoma-shi, Nara-ken (JP)
(74) Representative: Luckhurst, Anthony Henry William

(56) References cited:
- EP-A- 0 529 681
- EP-A- 0 661 008
- EP-A- 0 719 507
- US-A- 3 862 634
- US-A- 5 605 729

## Description

### BACKGROUND OF THE INVENTION

### 1. Field of the Invention

This invention relates to a book or loop type fastening device to be used for holding a disposable diaper of any known type onto a body of a wearer and also to a disposable diaper equipped with such fastening devices. More particularly, the present invention relates to a fastening device that can be manufactured at low cost with high productivity and is adapted to be fitted to a diaper with ease and keep the diaper in a folded state after use to make the diaper ready for waste disposal. The present invention also relates to a disposable diaper equipped with such fastening devices.

### 2. Prior Art

In recent years, disposable diapers mainly made of nonwoven textile or paper have been widely used for those who need diapers regardless of age. Paper diapers are most popular among babies and patients in hospitals. Such a type of diaper generally has a composite structure comprising top and bottom sheet members, a liquid-absorbing layer arranged between the top and bottom sheet members, and a fastener for holding the diaper to the body of the wearer.

Meanwhile, users of disposable diapers have a number of requirements to be satisfied by such diapers, including functional requirements to be met by the product per se such as a high liquid absorbing capacity of the liquid-absorbing layer, a high air permeability, a good anti-leakage effect for preventing liquid from flowing out of the diaper and an agreeable touch. It is also required that the diaper be put on and off the body with ease, it be not come off in use and that it be held in a small stable folded state for waste disposal with excretion contained therein so that it can be handled in a good sanitary condition.

Proposals have been made to meet such requirements that the diaper be put on and off with ease but it be not come off in use and that it be held in a small stable folded state for waste disposal with excretion contained in it.

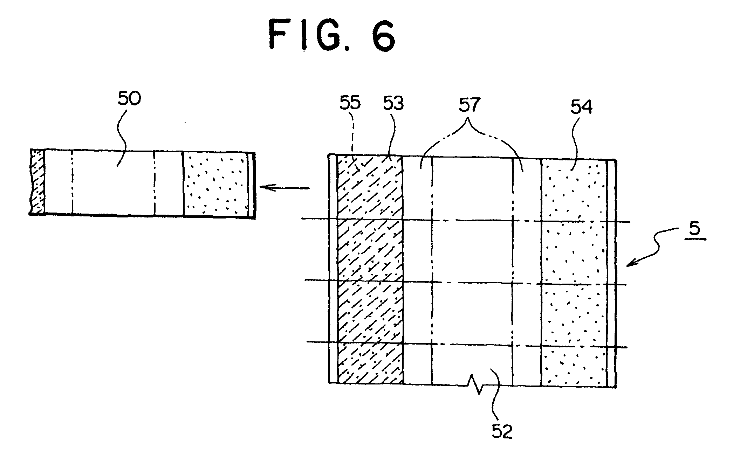

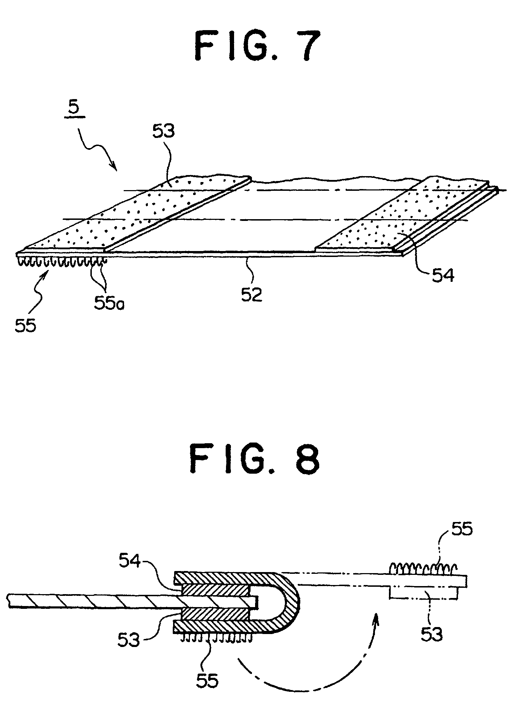

For example, Japanese Patent Laid-Open publication No. 2-5946 describes a disposable diaper that is characterized by specifically designed tape-shaped fasteners fitted to the diaper, as shown in FIGS. 6 and 7 of the Publication. Each of the tape-shaped fastener is secured at an end thereof to a diaper main body while its free end has a male engaging surface carrying thereon a plurality of hook engaging members releasably engageable with an area of a front surface of the diaper that carries thereon engaging loops. On the other hand, a pressure-sensitive adhesive layer is arranged on a surface of the tape, toward which the free end of the tape is folded, at a middle of the tape in the longitudinal direction thereof. With the above described arrangement, the free end of the tape-shaped fastener is folded so as to expose the male engaging surface with its back surface pressed and adhered to the pressure-sensitive adhesive layer. For disposing the diaper as waste, after the used diaper main body is rolled up or folded the free end portions of the tape-shaped fasteners are unfolded to become straight and peeled off the pressure-sensitive adhesive layer so that the pressure-sensitive adhesive layer may be pressed and adhered to a surface of the rolled diaper main body to keep it in that state and ready for disposal.

Japanese Utility Model Laid-Open Publication No. 6-61227 describes another tape-shaped fastener similar to the above described one but carries a pressure-sensitive adhesive layer both at one end portion and at the middle of the same surface of each of the tape-shaped fasteners. A plurality of hook engaging members having a structure as mentioned above are arranged on the same surface between the two press-sensing layers. With this arrangement, the one end portion of the tape carrying thereon a pressure-sensitive adhesive layer is pressed and adhered to the diaper main body while the other end portion of the tape-shaped fastener carrying neither an adhesive layer nor an engaging surface is folded and pressed against the pressure-sensitive adhesive layer arranged at the middle of the tape-shaped fastener for normal use. For disposing the diaper as waste, the used diaper main body is rolled up or folded and then the covering end of the tape-shaped fastener is peeled off the pressure-sensitive adhesive layer to expose the adhesive layer, which is pressed and adhered to the rolled diaper main body, so that the diaper can be ready for disposal.

However, in the fastener of a disposable diaper disclosed in the above-mentioned Japanese Patent Laid-Open Publication No. 2-5946, at least the pressure-sensitive adhesive layer formed on each of the tape-shaped fasteners is fitted to the diaper main body. Therefore, if an independent surface fastener is used to realize the surface carrying thereon engaging hook members, although the Publication does not describe how such a surface fastener is fitted to the diaper main body, it will have to be secured to the tape-shaped fastener by sewing or by fusing or adhesive-bonding before the tape is fitted to the diaper main body. In either case that the surface fastener is integrally formed with the tape-shaped fastener or they are prepared separately and then put together, the tape-shaped fastener carries thereon a pressure-sensitive adhesive layer and has to be handled with thorough care because the pressure-sensitive adhesive layer can loose its bonding effect, for example, by nursery powder adhering to it.

Meanwhile, engaging hook members as small as microns are used for such surface fasteners applied to diapers in recent years. Such engaging hook members have only small engaging strength that will be rapidly decreased after repeated use. Thus, the surface fasteners can be used only for a limited number of times. However, since the surface fastener of a disposable diaper disclosed in the above Publication is integrally formed with a tape rigidly secured to the diaper, it cannot be replaced with a new one.

On the other hand, the tape-shaped fastener of a disposable diaper disclosed in Japanese Utility Model Laid-Open Publication No. 6-61227 can be replaced and exchanged with a new one because it is secured to the diaper main body by means of a pressure-sensitive adhesive layer. However, replacing such a tape-shaped fastener is costly and can constitute a fatal disadvantage for a disposable diaper that should be made commercially available at low cost. Additionally, the fastener of a disposable diaper as disclosed in the above Publication is required to have a length sufficient for its free end portion to cover a pressure-sensitive adhesive layer, which would require the tape to have a more length than necessary for normal operation of itself.

In view of the above-mentioned problems of the known fasteners of disposable diapers, it is therefore an object of the present invention to provide a structurally simple and exchangeable fastening device of a disposable diaper that can be manufactured at low cost and is adapted to be fitted to a free end portion of a corresponding tape of the diaper with ease and keep the diaper in a folded state after use to make the diaper ready for waste disposal. Another object of the present invention is to provide a disposable diaper equipped with such fastening devices.

### SUMMARY OF THE INVENTION

According to a first aspect of the invention, the above object is achieved by providing a fastening device, or a surface fastener, to be fitted to a free end portion of each of a pair of tapes extending from lateral side edges of one longitudinal end portion of a disposable diaper comprising a strip-shaped substrate, characterized by first and second pressure-sensitive adhesive layers arranged at longitudinal opposite end portions of one surface of the substrate and a male or female engaging surface area arranged on the other surface of the strip-shaped substrate at a position opposite to the first pressure-sensitive adhesive layer.

For fitting the fastening device to a disposable diaper, the strip-shaped substrate is folded into halves to pinch the free end portion of the corresponding one of the tapes, in such a manner that the first and second pressure-sensitive adhesive layers arranged at the longitudinal opposite end portions of the strip-shaped substrate face each other via the free end portion of the corresponding tape and that the male or female engaging surface area are directed to a companion engaging surface area so that the fastening device may be releasably secured to the tape by way of the pressure-sensitive adhesive layers.

A fastening device having a structure as described above may be combined for shipment with a disposable diaper having a pair of corresponding tapes that are secured thereto by ordinary attaching means as components of the fastening means of the diaper. It is needless to say that the pressure-sensitive adhesive layers of the fastening devices in that state are covered by a separating paper. A user who purchased such a disposable diaper in combination with a pair of fastening devices may simply peel the separating paper off each of the fastening devices and secure the fastening devices to the disposable diaper by pressing and adhering them to the free end portions of the corresponding fastening tapes of the diaper, thus the assembling operation is completed. If the disposable diaper is of a reusable type and the engaging strength of the engaging elements is reduced by repeated use, the fastening devices may be removed from the corresponding tapes and replaced and exchanged with new ones.

Preferably, a fastening device according to the invention is produced by cutting a long primary product comprising a long and flexible substrate to be cut transversely into strip-shaped substrates, each having a predetermined length equal to a width of the long substrate, first and second pressure-sensitive adhesive layers continuously arranged on one surface of the long substrate along widthwise ends thereof, and a continuous male or female engaging surface area formed on the other surface opposite to the one surface at a position opposite to the first pressure-sensitive adhesive layer. Therefore, the fastening devices can be manufactured highly efficiently to reduce the manufacturing cost.

Preferably, said strip-shaped substrate and the engaging members of said engaging surface area are made of thermoplastic synthetic resin material and formed integrally with each other. While an ordinary injection molding process may be used for integrally producing such a strip-shaped substrate and such engaging members, a continuous molding method as disclosed in FIGS. 1 and 2 of Japanese Patent Laid-Open Publication No. 8-174693 or in Japanese Patent Publication Laid-Open No. 7-184707 may be preferable. Such a continuous molding method is particularly efficient for the purpose of the invention because a pressure-sensitive adhesive agent can be continuously applied to the widthwise end portions of the long substrate in a step following the molding process.

While a disposable diaper according to the invention may be handled exactly same as any known disposable diapers, a disposable diaper according to the invention that has been used can be rolled up or folded up in a small stable manner without allowing the excretion contained in it to leak out, so that it may be disposed in a good sanitary fashion. For example, a disposable diaper that has been used may be folded in by a 1/3 to 2/5 of its whole rear part thereof and then rolled up toward its front part. At that time, the pressure-sensitive adhesive layer of each of the fastening devices to be adhered to the rolled end of the diaper is peeled off from the corresponding fastening tape that is extending from the corresponding lateral side edge of the one longitudinal end portions of the diaper. Then each of the fastening tapes is folded toward the rolled end of the diaper so that the rolled up diaper may keep its state as the exposed pressure-sensitive adhesive layer is pressed and adhered to an exposed surface of the diaper.

### BRIEF DESCRIPTION OF THE DRAWINGS

FIG. 1 is a schematic perspective view of a typical embodiment of a disposable diaper according to the invention in an unused state.
FIG. 2 is a schematic perspective view of the disposable diaper of FIG. 1, showing how it is folded for waste disposal.
FIG. 3 is a schematic lateral view of a fastening device according to the invention that can be used for the disposable diaper of FIG. 1.
FIG. 4 is a schematic perspective view of the fastening device of FIG. 3.
FIG. 5 is a schematic lateral view showing a process for manufacturing the fastening device by molding according to the invention.
FIG. 6 is a schematic partial plan view of a product molded by the manufacturing process of FIG. 5.
FIG. 7 is a schematic partial perspective view of the product molded by the manufacturing process of FIG. 5.
FIG. 8 is a schematic lateral cross sectional view of a tape equipped with the fastening device according to the invention.
FIGS. 9A through 9E show steps of folding a disposable diaper for waste disposal according to the invention.

### DETAILED DESCRIPTION OF PREFERRED EMBODIMENTS

Now, preferred embodiments of the present invention will be described in greater detail by referring to the accompanying drawings.

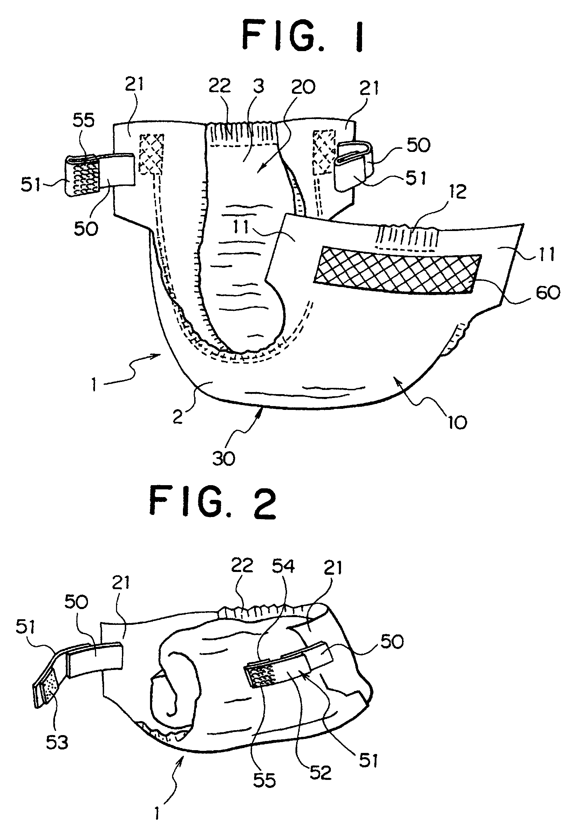

FIG. 1 is a schematic perspective view of a typical embodiment of a disposable diaper according to the invention in an unused state, and FIG. 2 is a schematic perspective view of the disposable diaper of FIG. 1, showing how it is folded for waste disposal. While a disposable diaper of this embodiment is designed for babies, it will be appreciated that those skilled in the art could modify it into a disposable diaper good for adults without departing from the scope of the invention.

A disposable diaper main body 1 of the diaper includes an front portion 10, a back portion 20 and a crotch portion 30. The entire diaper main body 1 comprises a bottom sheet 2 facing outside when fitted to the wearer and a top sheet 3 to be held in contact with a skin of a wearer and a liquid-absorbing layer (not shown) arranged between the bottom seat 2 and the top sheet 3. The bottom seat 2 and the top sheet 3 have a substantially identical contour, which has a substantially H-shape with a first pair of wings 11, 11 and a second pair of wings 21, 21 extending laterally of the diaper when the two sheets are put together to form a multilayer structure. A liquid-absorbing layer is arranged between the bottom seat 2 and the top sheet 3 over an entire area of the diaper except the peripheral edges of the bottom seat 2 and the top sheet 3 and the first and second pairs of wings 11, 11, 21, 21. The bottom seat 2 and the top sheet 3 are bonded together along the periphery thereof by means of adhesive or high frequency fusion-bonding.

The bottom seat 2 has to be water-resistant. While it may be simply made of synthetic resin film of a material such as polyethylene or polyolefin, it is more preferable that its outer surface provides a touch of woven fabric. For example, the film is preferably subjected to a finely embossing process or it may alternatively be laid integrally on the rear surface of a sheet of unwoven fabric. When laying the film on a sheet of unwoven fabric to form a multilayer structure, they may be bonded together at a large number of spots or stripes by means of adhesive-, high frequency fusion- or thermal fusion-bonding. Preferably, said film is not only water-resistant but also permeable to air.

The top sheet 3 may be made of either a hydrophilic material or a hydrophobic material and should be permeable to water. From the economic point of view, it is preferably made of unwoven fabric of synthetic fibers. Synthetic fibers that can be used for that purpose include polyethylene fibers, polypropylene fibers, polyester fibers and nylon fibers. The top sheet 3 made of such synthetic fibers may be made more flexible. For example, it may be made of unwoven fabric of crimped composite fibers spun out of two different synthetic resin materials having different shrinkage percentages with respect to boiling water that are bonded together and treated by boiling water to make them crimped. More specifically, such unwoven fabric may be manufactured by uniformly adding powder of synthetic resin material to a mass of fibers at a predetermined rate to produce webs, the synthetic resin having a melting point lower than that of the fibers. Then, the webs are heated by temperature higher than the melting point of the synthetic resin material but lower than the fibers, so that the synthetic resin powder are melted to gather at the crossings of fibers by the capillary phenomenon. As a result, the fibers may become bonded together to turn themselves into unwoven fabric when cooled. With a more simple technique, unwoven fabric may be produced by uniformly spraying adhesive agent to the webs of the fibers at a predetermined rate. In this case, a diluent are added to the adhesive agent.

The liquid-absorbing layer is typically made of a web of synthetic hydrophilic fibers such as cellulose fibers or polyvinylalcohol fibers, to which granules or fibers of highly liquid-absorbing material prepared from gel material may be added. Examples of gelled highly liquid-absorbing materials that can be used for that purpose include inorganic materials such as silica gel -and organic constitutions such as cross-link polymers. Preferably, the liquid-absorbing layer has a multilayer structure of several thin film layers of fiber webs to improve its ability of retaining liquid and, at the same time, holding the highly liquid-absording material in a stable manner in the inside thereof.

Further, this embodiment has major characteristic features as described below. Each of the second wings 21, 21 extending laterally from the opposite ends of a waist-fit portion 22 of the back portion 20 has a fastening tape 50 at the free end thereof. A abdominal-fit portion 12 of the front portion 10 carries on an outer surface thereof a female surface fastener area 60 extending over the entire width thereof.

According to the invention, the fastening tape 50 may be made of woven cloth, knit, unwoven fabric or a synthetic resin sheet or film. From an economical point of view, the use of unwoven fabric or a synthetic resin sheet or film is preferable. The fastening tape 50 is secured at an end to the front end of the corresponding second wing 21 by known means such as sewing, thermal fusing, ultrasonic fusing or high frequency fusing.

In this embodiment, the fastening tapes 50 are made of thermoplastic synthetic resin material. Their front and rear surfaces are not formed or molded in a specific way but are simply flat.

The most characteristic component of a disposable diaper according to the invention is a "fastening device". This fastening device is securely bonded to a free end portion of a corresponding one of the fastening tapes 50 of the diaper by means of the pressure-sensitive adhesive layer and peeled off from the fastening tape 50 whenever necessary.

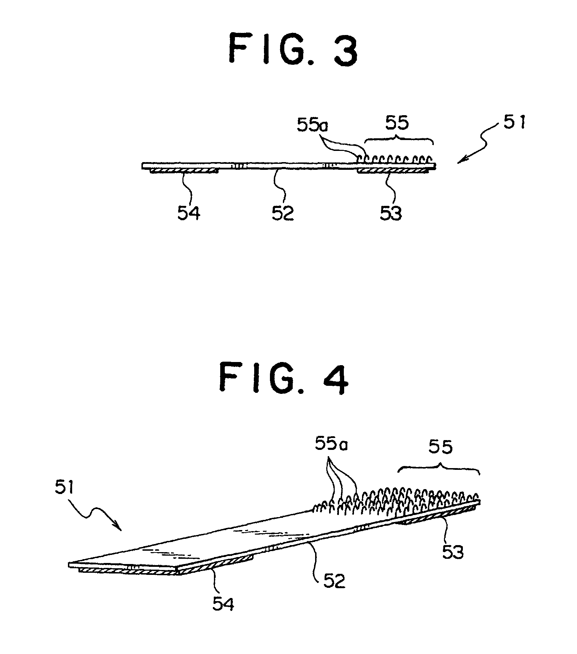

Now, a structure of each of the fastening devices 51 will be described by referring to FIGS. 3 and 4. The fastening device 51 is strip-shaped and has the same width as that of the fastening tape 51. It comprises a strip-shaped substrate 52, a first pressure-sensitive adhesive layer 53, a second pressure-sensitive adhesive layer 54, the first and second pressure-sensitive adhesive layers 53, 54 being separated from each other and arranged at the longitudinal opposite end portions of one surface of the strip-shaped substrate 52, and an engaging surface area 55 arranged on the other surface opposite to either the first or the second pressure-sensitive adhesive layer 53, 54 to operate as surface fastener, the engaging surface area 55 carrying thereon a large number of hook-shaped engaging members 55a standing from the other surface of the strip-shaped substrate 52 and having substantially the same size as one of the oppositely disposed pressure-sensitive adhesive layers 53, 54.

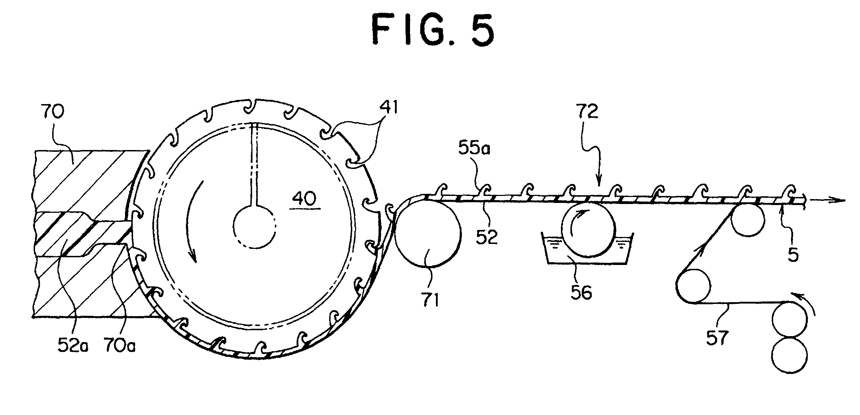

In this embodiment, the strip-shaped substrate 52 and the hook-shaped engaging members 55a are made of thermoplastic resin material such as polyamide, polyester or polypropylene like the fastening tapes 50 and formed integrally with each other. While they may be molded by means of a general injection molding process, preferably, they may be molded by a means as shown in FIG. 5. In the process, a cylinder 40 having a large number of hook-molding cavities 41 arranged along a peripheral surface thereof is rotated constantly in one direction, and at the same time, molten thermoplastic synthetic resin 52a is extruded from a die 70 of an extruder toward the peripheral surface of the cylinder 40 to fill the hook-molding cavities 41 and also a gap between an extrusion orifice 70a of the extruder and the outer peripheral surface of the cylinder 40. The gap is substantially equal to a thickness of the strip-shaped substrate 52 to be produced.

The process of molding the substrate will be described briefly. As molten synthetic resin is extruded toward the outer peripheral surface of the rotating cylinder 40, the hook molding cavities 41 are directly filled with molten synthetic resin 52a to produce hook-shaped engaging members 55a and, at the same time, a substrate 52 in the gap between the extrusion orifice 70a and the outer peripheral surface of the cylinder, which is moved along the direction of rotation of the cylinder 40. The molded product comprising a long substrate 52 and hook-shaped engaging members 55a is made to rotate around the outer peripheral surface of the cylinder 40 by a half turn thereof as they are cooled by an appropriate cooling means before they are continuously taken up by a take-up rollers 71. Thereafter, the molded product is fed to a coating step 72 where a pressure-sensitive adhesive agent 56 is applied to the lateral ends of the surface of the substrate 52 opposite to the surface where the engaging surface area 55 is formed. If necessary, separating papers 57 are continuously fed onto the surface of the substrate 52 carrying thereon the pressure-sensitive adhesive agent 56 as indicated by phantom lines in FIG. 6.

FIGS. 6 and 7 schematically illustrate a molded product 5 carrying thereon the pressure-sensitive adhesive agent 56. Each of the fastening devices 51 of this embodiment is obtained by cutting the molded product 5 along a alternate long and short dash line in FIG. 6 running perpendicularly relative to a longitudinal direction of the molded product 5 to make each of the fastening devices 51 show a predetermined width.

The strip-shaped substrate 52 and the engaging surface area 55 of the fastening device 51 may be made of cloth or knit. In this case, monofilaments for producing male engaging members are woven or knitted into a textile to make piles by means of a known process, and thereafter some of the piles are cut and heat set to produce hook-shaped engaging members 55a as shown in FIG. 8. Additionally, the top of each cut piles may be molten to make it show a mushroom-like profile.

On the other hand, a strip-shaped female surface fastener 60 is fitted to a center of the waist-fit portion 12 of the front portion 10. In this embodiment, the female surface fastener 60 is produced independently and firmly bonded to a center of the waist-fit portion 12 of the diaper main body 1. While the female surface fastener 60 may be made of piled woven or knit cloth, it may be replaced with a piece of unwoven fabric formed at the center of the waist-fit portion 12 of the bottom seat 2 if the male engaging members 55a of the companion fastening tape 50 have a fine and delicate profile, which can realize a reliable engagement.

A disposable diaper according to the invention is completed when the pressure-sensitive adhesive layers 53, 54 of a pair of fastening devices 51 are pressed and adhered to the respective free end portions of the respective fastening tapes 50 extending from the diaper main body 1 as shown in FIGS. 1, 2 and 8. More specifically, the separating paper 57 is removed from the pressure-sensitive adhesive layers 53, 54 of each of the fastening devices 51, and the pressure-sensitive adhesive layers 53, 54 are made to face each other by turning the fastening device 51 as illustrated by a solid line in FIG. 8 so as to pinch the free end portion of the corresponding fastening tape 50. Thus, the fastening device 51 is firmly fitted to the fastening tape 50 as the opposite ends of the fastening device 51 are pressed and adhered to the free end portion of the fastening tape 50.

According to the invention, the above operation of bonding each fastening device 51 to a corresponding fastening tape 50 can be carried out when the disposable diaper is shipped from the factory or from the distributor or after the end user actually uses, which is a matter of choice. Further, what is very characteristic about the invention is that the fastening devices of a disposable diaper according to the invention are exchangeable, taking an advantage of the use of the pressure-sensitive adhesive agent. While disposable diapers may more often than not be used only for once, there are disposable diapers adapted to be used repeatedly. In this case, such disposable diapers have to be made of washable materials. However, with known such disposable diapers comprising engaging members, the engaging elements are more apt to be worn out to reduce the engaging effect of the surface fastener earlier than the diaper main body 1.

To the contrary, however, if the fastening devices 51 of a disposable diaper according to the invention are worn out in the course of repeated use, they may simply be removed from the fastening tapes 50 and replaced by spares to make the disposable diaper reusable.

A disposable diaper according to the invention and having a structure as described above can be used just as any ordinary known disposable diapers and hence how to use it will not be described here any further. On the other hand, it is often cumbersome to dispose a used disposable diaper with excretion contained in it. However, a disposable diaper according to the invention and having a configuration as described above can be folded in a simple manner and held in a small folded state without allowing the internal excretion to leak out. Thus, the diaper can be disposed as waste in a highly sanitary fashion.

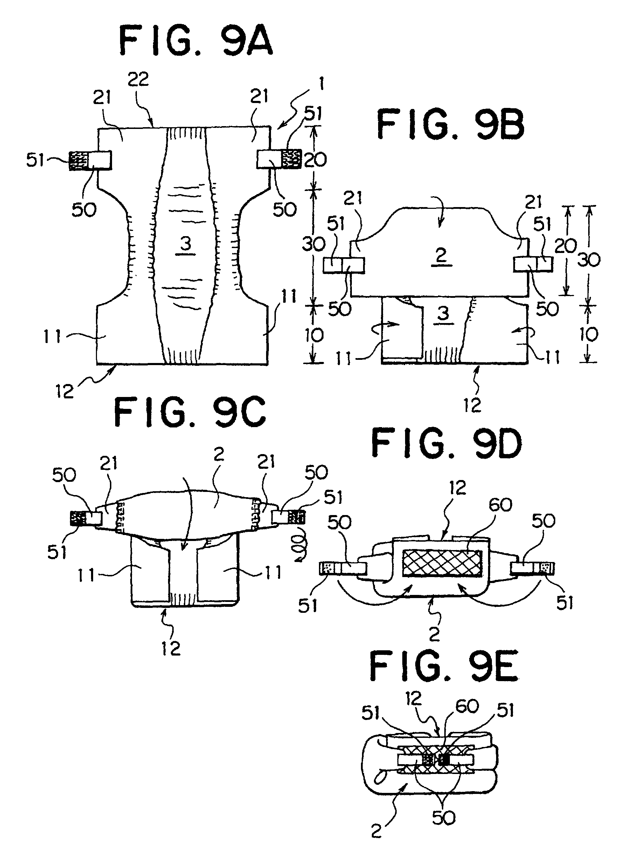

Now, a preferred method of folding a disposable diaper of the above-described embodiment will be described briefly by referring to FIGS. 9A through 9E, which show the steps of folding the disposable paper diaper. Firstly, as shown in FIG. 9A, the back portion 20 of the unfolded diaper main body 1 with the top sheet facing upward is folded upward to entirely cover the crotch portion 30 and then the first wings 11, 11 extending laterally from the front portion 10 are folded onto the center of the front portion 10 as shown in FIG. 9B. Then, the back portion 20 is rolled up in a direction indicated by an arrow in FIG. 9C for several turns to make the diaper appear as in FIG. 9D. At this time, the fastening tapes 50 are extending from the lateral sides of the rolled diaper main body 1 together with the respective fastening devices 51 carrying a large number of engaging members 55a on each of its surfaces, while the female surface fastener 60 on the front portion 10 is exposed on an surface of the rolled diaper main body as shown FIG. 9E.

Then, the first pressure-sensitive adhesive layer 53 of each of the fastening device 51 is peeled off the corresponding fastening tape 50 in a manner as indicated by a phantom arrow in FIG. 8, and then the fastening devices 51 and the fastening tapes 50 are folded as indicated by the arrow in FIG. 9D, so that the diaper main body 1 can maintain its rolled state when the peeled-off first pressure-sensitive adhesive layers 53, 53 of the fastening devices 51 are pressed and adhered to the diaper main body 1 as shown in FIG. 9E.

## Claims

1. A fastening device to be fitted to a free end portion of each of a pair of tapes (50) extending from lateral side edges of one longitudinal end portion of a disposable diaper, comprising a strip-shaped substrate (52), **characterized in that** first and second pressure-sensitive adhesive layers (53, 54) arranged at the longitudinal opposite end portions of one surface of the substrate (52) and a male or female engaging surface area (55) arranged on the other surface of the strip-shaped substrate (52) opposite to said one surface at a position opposite to said first pressure-sensitive adhesive layer (53).

2. A fastening device according to claim 1 **characterized in that** the fastening device (51) is formed by cutting a long primary product comprising a long and flexible substrate (52) to be cut into strip-shaped substrates (52), each having a predetermined length equal to a width of the long substrate (52), first and second pressure-sensitive adhesive layers (53, 54) continuously arranged on one surface of the long substrate along widthwise ends thereof, and a continuous male or female engaging surface area formed on the other surface opposite to said one surface along one of the widthwise ends of the long substrate (52) opposite to said first pressure-sensitive adhesive layer (53).

3. A fastening device according to claim 1 or 2, **characterized in that** said strip-shaped substrate (52) and engaging members (55a) of said engaging surface area are made of thermoplastic synthetic resin material and formed integrally with each other.

4. A fastening device according to claim 1, **characterised in that** it is used in a disposable diaper equipped with a pair of tapes (50) extending from lateral side edges of one longitudinal end portion of a disposable diaper main body (1) of the diaper.

5. A fastening device according to claim 1, **characterized in that** it is folded into halves to pinch the free end portion of the corresponding one of the tapes (50) of the disposable diaper, in such a manner that the first and second pressure-sensitive adhesive layers (53, 54) formed at the longitudinal opposite end portions of the strip-shaped substrate (52) of each of the fastening devices (51) are arranged to face each other via the free end portion of the corresponding tape (50) and that the engaging surface area (55) is arranged to face a corresponding engaging surface area (60) so that the fastening devices (51) can be releasably secured to the tapes (50) by means of said pressure-sensitive adhesive layers (53, 54).

## Patentansprüche

1. Verschlussvorrichtung, die an einem freien Ende jedes einzelnen eines Paares von Bändern (50) anzubringen ist, die sich von den Seitenkanten des einen Längsendes einer Wegwerfwindel aus erstrecken, wobei sie ein streifenförmiges Trägermaterial (52) aufweist und **dadurch gekennzeichnet ist, dass** erste und zweite selbstklebende Klebstoffschichten (53, 54) an in Längsrichtung entgegengesetzten Enden der einen Fläche des Trägermaterials (52) angeordnet sind und ein eindringende oder aufnehmende Eingreifelemente aufweisendes Flächenhaftverschlussgebiet (55) auf der anderen Fläche des streifenförmigen Trägermaterials (52) angeordnet ist, und zwar gegenüberliegend der einen Fläche und bei einer der ersten selbstklebenden Klebstoffschicht (53) gegenüberliegenden Position.

2. Verschlussvorrichtung nach Anspruch 1, **dadurch gekennzeichnet, dass** die Verschlussvorrichtung (51) durch Zerschneiden eines langen Primärproduktes ausgebildet ist, welches ein langes und flexibles Trägermaterial (52) beinhaltet, das in streifenförmige Trägermaterialstücke (52) zu zerschneiden ist, von denen jedes eine vorbestimmte Länge hat, die der Breite des langen Trägermaterials (52) entspricht, erste und zweite selbstklebende Klebstoffschichten (53, 54), welche auf der einen Seite des langen Trägermaterials an dessen Querrichtungsenden zusammenhängend angeordnet sind, und ein zusammenhängendes, eindringende oder aufnehmende Eingreifelemente aufweisendes Flächenhaftverschlussgebiet aufweist, das auf der anderen Fläche ausgebildet ist, die der einen Fläche gegenüberliegt, und zwar entlang eines der Querrichtungsenden des langen Trägermaterials (52) und gegenüberliegend der ersten selbstklebenden Klebstoffschicht (53).

3. Verschlussvorrichtung nach Anspruch 1 oder 2, **dadurch gekennzeichnet, dass** das streifenförmige Trägermaterial (52) und die Eingreifelemente (55a) des Flächenhaftverschlussgebietes aus thermoplastischem Kunstharzmaterial bestehen und integral miteinander ausgebildet sind.

4. Verschlussvorrichtung nach Anspruch 1, **dadurch gekennzeichnet, dass** sie bei einer Wegwerfwindel verwendet wird, welche mit einem Paar Bänder (50) ausgerüstet ist, die sich von den Seitenkanten des einen Längsendes des Hauptkörpers (1) einer Wegwerfwindel aus erstrecken.

5. Verschlussvorrichtung nach Anspruch 1, **dadurch gekennzeichnet, dass** sie auf halbe Größe aufeinandergefaltet ist, so dass das freie Ende des zugehörigen der Bänder (50) der Wegwerfwindel eingeklemmt wird, derart, dass die ersten und zweiten selbstklebenden Klebeschichten (53, 54), die an den in Längsrichtung gegenüberliegenden Enden des streifenförmigen Trägermaterials (52) von jeder der Befestigungsvorrichtungen (51) ausgebildet sind, so angeordnet sind, dass sie einander, mit dem dazwischenliegenden freien Ende des zugehörigen Bandes (50), zugewandt sind und dass das Flächenhaftverschlussgebiet (55) so angeordnet ist, dass es einem zugehörigen Flächenhaftverschlussgebiet (60) zugewandt ist, so dass die Verschlussvorrichtungen (51) mittels der selbstklebenden Klebstoffschichten (53, 54) lösbar an den Bändern (50) befestigt werden können.

## Revendications

1. Dispositif de fixation destiné à être ajusté sur une partie d'extrémité libre de chacune d'une paire de bandes (50) s'étendant à partir des bords des côtés latéraux d'une partie d'extrémité longitudinale d'une couche jetable, comprenant un substrat en forme de ruban (52), **caractérisé par** des première et deuxième couches autoadhésives (53, 54) agencées au niveau des parties d'extrémité longitudinales opposées d'une surface du substrat (52) et une aire de surface d'engagement mâle ou femelle (55) agencée sur l'autre surface du substrat en forme de ruban (52) opposée à ladite une surface, au niveau d'une position opposée à ladite première couche autoadhésive (53).

2. Dispositif de fixation selon la revendication 1, **caractérisé en ce que** le dispositif de fixation (51) est formé en découpant un produit primaire long comprenant un substrat long et flexible (52) destiné à être découpé en des substrats en forme de ruban (52), ayant chacun une longueur prédéterminée égale à une largeur du substrat long (52), des première et deuxième couches autoadhésives (53, 54) agencées en continu sur une surface du substrat long le long des extrémités correspondantes dans le sens de la largeur, et une aire de surface d'engagement mâle ou femelle formée sur l'autre surface opposée à ladite une surface le long de l'une des extrémités dans le sens de la largeur du substrat long (52) opposée à ladite première couche autoadhésive (53).

3. Dispositif de fixation selon les revendications 1 ou 2, **caractérisé en ce que** ledit substrat en forme de ruban (52) et les éléments d'engagement (55a) de ladite aire de surface d'engagement sont composés d'une résine thermoplastique synthétique et formés d'une seule pièce les uns avec les autres.

4. Dispositif de fixation selon la revendication 1, **caractérisé en ce qu'**il est utilisé dans une couche jetable équipée d'une paire de bandes (50) s'étendant à partir des bords des côtés latéraux d'une partie d'extrémité longitudinale d'un corps principal de couche jetable (1) de la couche.

5. Dispositif de fixation selon la revendication 1, **caractérisé en ce qu'**il est plié en deux moitiés pour serrer la partie d'extrémité libre d'une des bandes correspondantes (50) de la couche jetable, de sorte que les première et deuxième couches autoadhésives (53, 54) formées au niveau des parties d'extrémité longitudinales opposées du substrat en forme de ruban (52) de chacun des dispositifs de fixation (51) sont agencées de sorte à se faire face par l'intermédiaire de la partie d'extrémité libre de la bande correspondante (50), l'aire de la surface d'engagement (55) étant agencée de sorte à faire face à une aire de surface d'engagement correspondante (60), les dispositifs de fixation (51) pouvant ainsi être fixés de manière amovible sur les bandes (50) par l'intermédiaire desdites couches autoadhésives (53, 54).
